# EUROPEAN PATENT SPECIFICATION

(11) **EP 4 297 711 B1**
(45) Date of publication and mention of the grant of the patent: **20.08.2025**
(21) Application number: 21708631.3
(22) Date of filing: 26.02.2021
(51) Int. Cl.: A61F 13/505

(54) **REUSABLE OUTER SHELL FOR AN ABSORBENT INSERT**
WIEDERVERWENDBARE AUSSENHÜLLE FÜR EINEN SAUGFÄHIGEN EINSATZ
ENVELOPPE EXTERNE RÉUTILISABLE POUR UN INSERT ABSORBANT

(43) Date of publication of application: 03.01.2024
(73) Proprietor: Essity Hygiene and Health Aktiebolag, 405 03 Göteborg (SE)
(72) Inventor: CORNELIUSSON, Helena, 405 03 GÖTEBORG (SE); THELIN, Cecilia, 405 03 GÖTEBORG (SE); JOHANSSON, Mattias, 405 03 GÖTEBORG (SE)
(74) Representative: Hoffmann Eitle
(86) International application number: PCT/EP2021/054847
(87) International publication number: WO 2022/179697

(56) References cited:
- EP-A1- 2 810 629
- US-A1- 2010 318 057
- US-A1- 2012 116 339
- US-A1- 2014 257 228
- US-A1- 2018 193 205

## Description

### TECHNICAL FIELD

The present disclosure pertains to a reusable outer shell for an absorbent insert. In particular, this disclosure pertains to a reusable outer shell comprising a front pocket and a rear pocket for receiving the absorbent insert.

### BACKGROUND OF THE INVENTION

The present disclosure generally relates to the field of reusable and washable diapers, and more particularly to a reusable outer shell adapted for receiving an absorbent insert, either a reusable absorbent insert or a disposable absorbent insert.

For economic and environmental reasons, parts of the absorbent article or the whole absorbent article may be washable and reusable. The absorbent article may for example include a washable outer shell which is intended to receive an absorbent insert which may be removably attached to the outer shell. The washable outer shell may be reused after soiling and removal of the absorbent insert by attaching a new absorbent insert to the outer shell. Attaching a new absorbent insert to the outer shell may be cumbersome, and a correct placement and attachment is important both for comfort and for leakage security of the absorbent article.

Disposable diapers, such as open diapers and pant-diapers, are generally provided with elasticized standing gather material along the leg openings and waist elastics in the front and rear portions along the absorbent insert outer edges, in order to prevent leakage. An absorbent article comprising a reusable outer shell and an absorbent insert may be more difficult to provide with elastics in direct connection with the absorbent insert. It is generally preferable to provide the outer shell with the elastic elements, since this may allow using absorbent inserts having a simpler construction resulting in less material consumption for the disposable parts.

Furthermore, washing of washable and reusable outer shells comprising hook materials for releasable fastening of the absorbent insert may lead to impaired functionality of hook material and undesired attachment of hook materials to other parts of the outer shell or to other items during washing. Undesired attachment of hook materials to other parts of the outer shell may have negative impact on the cleaning of the outer shell and attachment to other items washed together with the outer shell may damage fragile textile materials.

There is thus still a need for a reusable outer shell which has improved leakage security and comfort, and which also has prolonged life span. There is also a need for a reusable outer shell which is easy and convenient to wash and which has an improved washing result.

US 2013/0318057 A1 discloses features falling under the preamble of claim 1. US 2012/116339 A1, US 2018/193205 A1, and EP 2 810 629 A1 are further prior art.

### SUMMARY OF THE INVENTION

One or more of the above objects may be achieved with a washable outer shell in accordance with claim 1, which defines the invention. Further embodiments are set out in the dependent claims and in the following description.

A reusable outer shell for a disposable absorbent insert according to the present disclosure comprises an inner surface intended to face the absorbent insert and an outer surface being opposite to the inner surface. The outer shell has an extension in the longitudinal direction and the transverse direction. The outer shell comprises a front region, a crotch region and a rear region, the outer shell comprising a front pocket in the front region and a rear pocket in the rear region. The front pocket is adapted to receive a front portion of the absorbent insert and the rear pocket is adapted to receive a rear portion of the absorbent insert, the front pocket having a front pocket transverse distal edge adapted to extend over the front portion of the absorbent insert and the rear pocket having a rear pocket transverse distal edge adapted to extend over the rear portion of the absorbent insert. The front pocket has a front pocket length, as measured along a longitudinal centerline of the outer shell from a front pocket transverse proximal edge to the front pocket transverse distal edge and the rear pocket has a rear pocket length, as measured along the longitudinal centerline of the outer shell and from a rear pocket transverse proximal edge to the rear pocket distal transverse edge, and wherein the rear pocket length is greater than the front pocket length.

The reusable outer shell is intended to be reused after soiling by removing of a soiled absorbent insert and by attaching a new absorbent insert to the reusable outer shell. Inserting a new absorbent insert to the outer shell may be cumbersome, and a correct placement and attachment is important both for comfort and for leakage security of the absorbent article. It has been found by the present inventors that by providing the outer shell with a front pocket arranged in the front region and a rear pocket arranged in the rear region the absorbent insert may be inserted into the outer shell and held in place both in the rear and front regions with a reduced risk of the absorbent insert being arranged obliquely in the outer shell with a resulting risk of side leakage.

It has furthermore been found by the present inventors that the absorbent insert was exerted to different types of stresses in the front region compared to in the rear region and that having a rear pocket with a greater length than a front pocket may provide the rear part of the absorbent insert with the stabilization needed to maintain a correct placement of the absorbent insert over the time of use.

Disposable diapers, such as open diapers and pant-diapers, are generally provided with elasticized standing gather material along the leg openings and waist elastics in the front and rear portions along the absorbent insert outer edges to prevent leakage. An absorbent article comprising two separate parts, as disclosed herein, may be more difficult to provide with elastics in direct connection with the absorbent insert. The provision of front and rear pockets to the outer shell according to the present disclosure were found to provide a protection from leakage in the front and rear regions since the pockets form front and rear leakage barriers.

The reusable outer shell is an outer shell that is capable of being reused multiple times by removing and replacing an absorbent insert with a new absorbent insert. The reusable outer shell may be a washable outer shell, capable of being washed without essential shrinkage or other damage, such as after five and up to forty washing occasions. The outer shell may made from a washable material such as a washable fabric. The outer shell may be made of one or more of the following materials: polyester, viscose, polyamide and/or cotton. The outer shell may be a stretchable outer shell, i.e., being made of a stretchable material. The outer shell may be an elastic shell, being elastic in the longitudinal and/or the transverse direction of the outer shell.

The rear pocket length may be at least 15% greater than the front pocket length, optionally at least 25% greater or at least 40% greater. This has been found to correspond to the increased stabilization needed in the rear portion of the absorbent insert and thereby provide a stable and reliable housing of the rear portion of the absorbent insert.

The rear pocket length may be 35 mm or more. Optionally, the rear pocket length may be from 35 mm to 100 mm. This has been found to provide a stable and reliable housing of the absorbent insert rear portion, providing leakage protection in the rear region of the outer shell, while not impairing the inlet properties of the absorbent insert.

The front pocket length may be 25 mm or more. Optionally, the front pocket length may be from 25 mm to 80 mm. This has been found to provide a stable and reliable housing of the absorbent insert front portion, while not impairing the inlet properties of the absorbent insert.

The rear pocket may have a rear pocket transverse distal edge region extending along the rear pocket transverse distal edge and an elastic member may be arranged in the rear pocket transverse distal edge region and extend in the transverse direction. This may facilitate a correct placement of the absorbent insert rear portion, in particularly over time. A further advantage is that the rear pocket transverse distal edge may rise with the elastic member arranged along the edge, thus enhancing the barrier function of the rear pocket.

The front pocket may have a front pocket transverse distal edge region extending along the front pocket transverse distal edge and an elastic member may be arranged in the front pocket transverse distal edge region and extend in the transverse direction. This may facilitate a correct placement of the absorbent insert front portion, in particularly over time. A further advantage is that the front pocket transverse distal edge may rise with the elastic member arranged along the edge, thus enhancing the barrier function of the front pocket.

The reusable outer shell comprises a front hook member arranged on the inner surface of the outer shell, inside the front pocket, the front hook member having a length extending in the longitudinal direction and a width extending in the transverse direction. To provide the reusable outer shell with a front hook member, arranged inside the front pocket, has been found to enhance the retention and correct placement in the outer shell over time of the absorbent insert.

The reusable outer shell may comprise a rear hook member arranged on the inner surface of the outer shell inside the rear pocket, the rear hook member having a length extending in the longitudinal direction and a width extending in the transverse direction. To provide the reusable outer shell with a rear hook member, arranged inside the rear pocket, has been found to enhance the retention and correct placement in the outer shell over time of the absorbent insert.

The length of the rear hook member may be greater than the length of the front hook member. The present inventors have found that the rear portion of the absorbent insert is exerted to higher shear and pull forces than the front hook member, particularly when applying the absorbent insert, but also during use. The rear portion of the absorbent insert is for example both during day and night-time exerted to shear forces resulting from when the user walks, sits and changes position, and move around during sleep. It has therefore been found beneficial to have a rear hook member with a greater length compared to the front hook member for keeping the absorbent insert in place during use and particularly over a longer period.

The length of the rear hook member may be at least 15% greater than the length of the front hook member, optionally at least 25% greater or at least 40% greater.

The length of the rear hook member may be 10 mm or more, optionally within the range of from 10 mm to 70 mm. This has been found to provide a satisfying attachment of the absorbent insert rear portion and maintain a correct positioning of the absorbent insert in the outer shell during use.

The length of the front hook member may be 5 mm or more, optionally within the range of from 5 mm to 50 mm. This has been found to provide a satisfying attachment of the absorbent insert front portion.

The width of the rear hook member may be within the range of from 50 mm to 200 mm. Optionally the width of the rear hook member may be within the range of from 75 mm to 150 mm.

The width of the front hook member may be within the range of from 50 mm to 200 mm. Optionally the width of the front hook member may be within the range of from 75 mm to 150 mm.

The reusable outer shell may be washed after use, such as machine washed. However, washing of the outer shell may lead to impaired functionality of the front and rear hook members. During washing of the washable outer shell, threads and other material debris may get caught into the hook material and thereby deteriorate the ability of the hook member to attach to the absorbent insert backside. A further issue with the outer shell being provided with hook members is that during washing, undesired attachment of the hook materials to other parts of the outer shell or to other items during washing may have negative impact on the cleaning of the outer shell and may damage fragile textile materials washed together with the washable outer shell. However, since the front and rear hook members are provided in a respective front and rear pocket, the outer shell according to the present disclosure provides a protection of the hook members as such and for undesired attachment of the hook members during washing.

The rear hook member may comprise a first rear hook member part and a second rear hook member part, the first rear hook member part and the second rear hook member part being spaced apart from each other with a distance and arranged on a respective side of a longitudinal centerline of the outer shell, the width of the rear hook member being measured at the greatest width between an outermost transverse edges of the respective first rear hook member part and second rear hook member part and the length of the rear hook member being measured at the greatest length between a frontmost edge of the first rear hook member part and the second rear hook member part and a rearmost edge of the respective first rear hook member part and second rear hook member part. Such configuration may allow attachment of the absorbent insert at a respective rear corner portion while using less hook material and increasing the stiffness in the rear region of the outer shell. Since the first rear hook member part and the second rear hook member part are arranged on a respective side of the longitudinal centerline, folding of the outer shell along the centerline is made easier.

The first rear hook member part may have the same width as the second rear hook member part. The first rear hook member part may, alternatively or additionally, have the same length as the second rear hook member part.

The first rear hook member part and the second rear hook member part may cover from 25% to 100% of an area of the rear hook member, as measured by multiplying the length of the rear hook member with the width of the rear hook member.

The first rear hook member part and the second rear hook member part may cover from 35% to 80% of an area of the rear hook member.

The distance between the first rear hook member part and the second rear hook member part may correspond to from 15% to 75% of the width of the rear hook member, as measured at the greatest distance.

The front hook member may comprise a first front hook member part and a second front hook member part, the first front hook member part and the second front hook member part being spaced apart from each other with a distance and being arranged on a respective side of a longitudinal centerline of the outer shell, the width of the front hook member being measured at the greatest width between the outermost transverse edges of the respective first front hook member part and second front hook member part and the length of the front hook member being measured at the greatest length between a frontmost edge of the first front hook member part and the second front hook member part and a rearmost edge of the first front hook member part and second front hook member part, optionally wherein the first front hook member part has the same width and/or length as the second front hook member part. Such configuration may allow attachment of the absorbent insert at a respective front corner portion while using less hook material and reducing the stiffness in the front region of the outer shell. Since the first front hook member part and the second front hook member part are arranged on a respective side of the longitudinal centerline, folding of the outer shell along the centerline is made easier.

The distance between the first front hook member part and the second front hook member part may correspond to from 15% to 75% of the width of the front hook member, as measured at the greatest distance.

The first front hook member part and the second front hook member part may cover from 25% to 100% of an area of the front hook member, as measured by multiplying the length of the front hook member with the width of the front hook member.

The first front hook member part and the second front hook member part may cover from 35% to 80% of an area of the front hook member.

The rear hook member may have a width to length ratio of from 0.7:1 to 20:1. Optionally, the rear hook member may have a width to length ratio of from 1.1:1 to 15:1.

The front hook member may have a width to length ratio of from 1:1 to 40:1. Optionally, the front hook member may have a width to length ratio of from 1.5:1 to 40:1.

The outer shell in the rear region may comprise a first hook member provided in a first rear transverse corner region on an inner surface of the outer shell and a second hook member provided in a second rear transverse corner region of the outer shell, the outer shell may comprise a hook attachment zone on the outer surface in the front region of the outer shell, the first and second hook members being adapted to be attached to the hook attachment zone to form a waist opening.

The outer shell may comprise leg elastics arranged along a respective longitudinal side edge of the outer shell in the crotch region and waist elastics arranged along a rear transverse edge in the rear region of the outer shell.

### BRIEF DESCRIPTION OF THE DRAWINGS

The present invention will be further explained hereinafter by means of non-limiting examples and with reference to the appended drawings wherein:
- Fig. 1A: illustrates a reusable outer shell according to the present disclosure as seen from the inner surface;
- Fig. 1B: illustrates the reusable outer shell from Fig. 1A as seen from the outer surface;
- Fig. 2: illustrates a reusable outer shell according to the present disclosure as seen from the inner surface; and,
- Fig. 3: illustrates the reusable outer shell according to Fig. 2 comprising an absorbent insert.

### DETAILED DESCRIPTION

The invention will be described more closely below by reference to an exemplary embodiment. The invention may however be embodied in many different forms and should not be construed as limited to the embodiments set forth in the drawings and the description thereto.

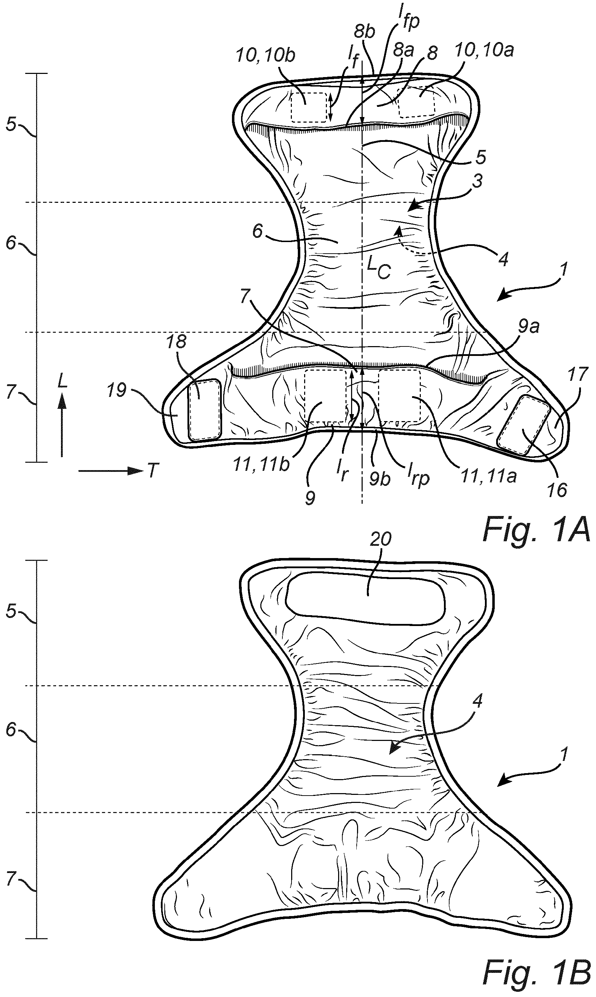

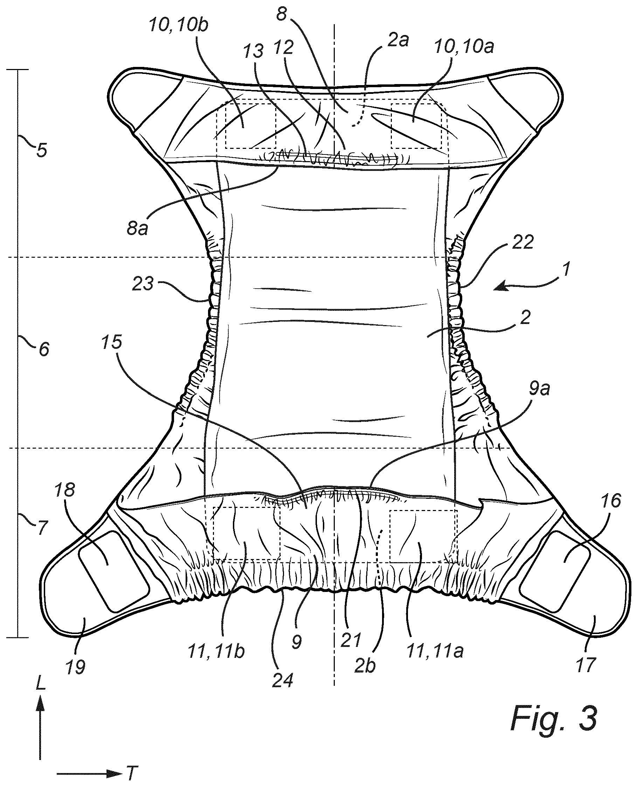

Figures 1A and 1B show a washable outer shell 1 according to the present disclosure. The outer shell 1 comprises an inner surface 3 and an outer surface 4 and has an extension in the longitudinal direction L and in the transverse direction T. Figure 1A is an illustration of the washable cover 1 as seen from the inner surface 3 and figure 1B shows the outer shell 1 as seen from the outer surface 4. The outer shell 1 shown in figures 1A and 1B is adapted to receive an absorbent insert 2 (shown in Fig. 3).

The reusable outer shell 1 is an outer shell that is capable of being reused multiple times. The reusable outer 1 shell may be a washable outer shell, capable of being washed without essential shrinkage or other damage. The outer shell 1 may be made from a washable material such as a washable fabric. The outer shell 1 may be made of one or more of the following materials; polyester, viscose, polyamide and/or cotton. The outer shell 1 may be a stretchable outer shell, i.e. being made of a stretchable material. The outer shell 1 may be an elastic shell, being elastic in the longitudinal direction L and/or the transverse direction T of the outer shell 1.

The outer shell 1 comprises a front region 5, a crotch region 6 and a rear region 7. The outer shell 1 comprises a front pocket 8 in the front region 5 and a rear pocket 9 in the rear region 7. The front pocket 8 has a front pocket length l_{fp}, as measured along a longitudinal centerline L_{c} of the outer shell 1 from a front pocket transverse proximal edge 8b to the front pocket transverse distal edge 8a and the rear pocket 9 has a rear pocket length lᵣₚ, as measured along the longitudinal centerline L_{c} of the outer shell 1 and from a rear pocket transverse proximal edge 9b to the inner rear transverse edge 9a. The rear pocket length lᵣₚ is greater than the front pocket length l_{fp}. The rear pocket length lᵣₚ may be at least 15% greater than the front pocket length l_{fp}. The rear pocket length lᵣₚ may be 35 mm or more and the front pocket length l_{fp} may be 25 mm or more.

The front pocket 8 and the rear pocket 9 may constitute of the same or different type of material as the outer shell 1. The pocket material may, as well as the outer shell material, be capable of being washed several times without essential shrinkage or other damage. The pockets may be made from a washable material such as a washable fabric. The pocket material may be a polyester, polyamid, cotton and/or a viscose material. The pocket material may be made of a stretchable material. The pocket material may be an elastic material, being elastic in the longitudinal direction L and/or the transverse direction T.

A front hook member 10 is arranged on the inner surface 3 of the outer shell 1, inside the front pocket 8 and a rear hook member 11 is arranged on the inner surface 3 of the outer shell 1 inside the rear pocket 9. The rear hook member 11 comprises a first rear hook member part 11a and a second rear hook member part 11b. The front hook member 10 comprises a first front hook member part 10a and a second front hook member part 10b. The first front hook member part 10a and the second front hook member part 10b are spaced apart from each other and are arranged on a respective side of a longitudinal centerline L_{c} of the outer shell 1. The first rear hook member part 11a and the second rear hook member part 11b are spaced apart from each other and are arranged on a respective side of a longitudinal centerline L_{c} of the outer shell 1. The front hook member 10 has a length l_{f} and the rear hook member has a length lᵣ, the respective lengths each extending in the longitudinal direction L. The length lᵣ of the rear hook member 11 is greater than the length l_{f} of the front hook member 10.

The front and rear hook members 10,11 may comprise any hook-type material with a hook surface structure and the terms hook members are to be interpreted as encompassing the hook part of a hook and loop fastening system, e.g., known as a VELCRO^{®} system. The "hooks" may have many different shapes which are adapted to engage with a loop part of the hook and loop fastening system. The hooks may, purely as an example, by of a J-shape, mushroom shape, and palm tree shape.

The outer shell 1 comprises a first hook member 16 in the rear region 7 provided in a first rear transverse corner region 17 on the rear pocket 9 and a second hook member 18 provided in a second rear transverse corner region 19 on the rear pocket, depending on the transverse extension of the pocket 9, the first and second hook members 16,18 may be provided on the rear pocket 9 or on the inner surface 3 of the outer shell 1.

The outer shell 1 comprises a hook attachment zone 20 on the outer surface 4 in the front region 5 of the outer shell 1, as illustrated in Fig. 1B. The first and second hook members 16,18 are adapted to be attached to the hook attachment zone 20 to form a waist opening.

The first and second hook members 16,18 may comprise any hook-type material with a hook surface structure and the hook attachment zone 20 may comprise any loop surface structure, and the terms hook members are to be interpreted as encompassing the hook part of a hook and loop fastening system, e.g., known as a VELCRO^{®} system. The "hooks" may have many different shapes which are adapted to engage with a loop part of the hook and loop fastening system. The hooks may, purely as an example, by of a J-shape, mushroom shape, and palm tree shape. The hook attachment zone 20 may comprise any type of loop surface structure, which is here to be understood to encompass a surface structure to which a hook material of a hook and loop fastening system is attachable. Accordingly, a loop surface structure may include fibers or threads extending from a surface and back into the surface to define genuine loops, as well as a surface structure including fibers or threads extending out from a surface and having loose ends, which fibers or threads entangle with each other. Upon attachment of the respective hook members 16,18, the hook surface structure engages with the loop surface structure providing a releasable attachment between the elements.

The outer shell 1 comprises leg elastics 22,23 arranged along a respective longitudinal side edge of the outer shell 1 in the crotch region 6 and waist elastics 24 arranged along a rear transverse edge in the rear region 7 of the outer shell 1.

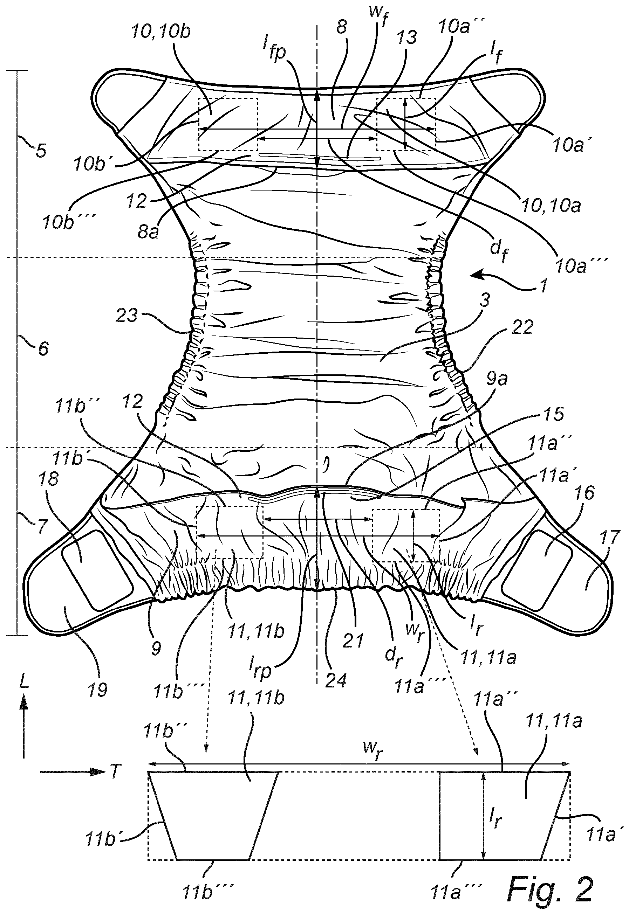

Fig. 2 illustrates a washable outer shell 1 according to the present disclosure. The outer shell 1 is illustrated as seen from an inner surface 3 and has an extension in the longitudinal direction L and in the transverse direction T. The outer shell 1 comprises a front region 5, a crotch region 6 and a rear region 7. The outer shell 1 comprises a front pocket 8 in the front region 5 and a rear pocket 9 in the rear region 7. The front pocket 8 has a front pocket length l_{fp}, as measured along a longitudinal centerline L_{c} of the outer shell 1 from a front pocket transverse proximal edge 8b to the front pocket transverse distal edge 8a and the rear pocket has a rear pocket length lᵣₚ, as measured along the longitudinal centerline L_{c} of the outer shell 1 and from a rear pocket transverse proximal edge 9b to the inner rear transverse edge 9a. The rear pocket length lᵣₚ is greater than the front pocket length l_{fp}. The rear pocket length lᵣₚ may be at least 15% greater than the front pocket length lᵣₚ. The rear pocket length lᵣₚ may be 35 mm or more and the front pocket length (l_{fp}) may be 25 mm or more.

A front hook member 10 is arranged on the inner surface 3 of the outer shell 1, inside the front pocket 8 and a rear hook member 11 is arranged on the inner surface 3 of the outer shell 1 inside the rear pocket 9. The rear hook member 11 comprises a first rear hook member part 11a and a second rear hook member part 11b. The front hook member 10 comprises a first front hook member part 10a and a second front hook member part 10b. The first front hook member part 10a and the second front hook member part 10b are spaced apart from each other with a distance d_{f} and are arranged on a respective side of a longitudinal centerline L_{c} of the outer shell 1. The first rear hook member part 11a and the second rear hook member part 11b are arranged on a respective side of a longitudinal centerline L_{c} of the outer shell 1.

The front hook member 10 has a length l_{f} extending in the longitudinal direction L and a width w_{f} extending in the transverse direction T. The length l_{f} is measured at the greatest length, in the longitudinal direction L, between a frontmost edge 10a",10b" of the first front hook member part 10a and the second front hook member part 10b and a rearmost edge 10a‴, 10b‴ of the first front hook member part 10a and second front hook member part 10b.

The rear hook member 11 has a length lᵣ extending in the longitudinal direction L and a width wᵣ extending in the transverse direction T. The length lᵣ is measured at the greatest length, in the longitudinal direction L, between a frontmost edge 11a",11b" of the first rear hook member part 11a and the second rear hook member part 11b and a rearmost edge 11a‴,11b‴ of the first rear hook member part 11a and second rear hook member part 11b.

The length lᵣ of the rear hook member 11 is greater than the length l_{f} of the front hook member 10.

The length of the of the front hook member 10 may be the same over at least 50% of the front hook member 10, as seen in the transverse direction T and the length of the of the rear hook member 11 may be the same over at least 50% of the rear hook member 11, as seen in the transverse direction T.

As for the measurements of the lengths of the hook members, the width wᵣ of the rear hook member 11 is measured, in the transverse direction T, at the greatest width between an outermost transverse edges 11a',11b' of the respective first rear hook member part 11a and second rear hook member part 11b. An alternative hook configuration is illustrated in Fig. 2 below the outer shell 1, wherein the first and second rear hook member parts 11a,11b each has a rhombus shape. The width wᵣ of the rear hook member 11 is thus not constant, as seen in the longitudinal direction L. The width wᵣ of the rear hook member 11 is measured by drawing an extension in the longitudinal direction L, at the outermost section of the respective outermost transverse edges 11a',11b' and measuring the width therebetween.

The first front hook member part 10a and the second front hook member part 10b cover from 25% to 100% of an area of the front hook member 10, as measured by multiplying the length l_{f} of the front hook member 10 with the width w_{f} of the front hook member 10.

The first rear hook member part 11a and the second rear hook member part 11b cover from 25% to 100% of an area of the rear hook member 11, as measured by multiplying the length lᵣ of the rear hook member 11 with the width wᵣ of the rear hook member 11.

In fig. 2, the first and the second front hook member parts have the same widths and the same length and are arranged with a distance d_{f} corresponding to from 15% to 75% of the width w_{f} of the front hook member 10, such as from 40% to 60% of the width w_{f} of the front hook member 10.

The front pocket 8 has a front pocket transverse distal edge region 12 extending along the front pocket transverse distal edge 8a and an elastic member 13 is arranged in the front pocket transverse distal edge region 12 and extends in the transverse direction T.

The rear pocket 9 has a rear pocket transverse distal edge region 15 extending along the rear pocket transverse distal edge 9a and an elastic member 21 is arranged in the rear pocket transverse distal edge region 15 and extends in the transverse direction T.

The elastic members 13,21 may be any suitable type of elastic members, such as for example a respective elastic thread, foam elastics or strips of an elastic film. The elastic members 13,21, may be of the same type of elastic material or of different types of elastic materials.

The rear region 7 of the outer shell 1 furthermore comprises a first corner region 17 and a second corner region 19. The outer shell 1 comprises a first hook member 16 provided in the first corner region 17 and a second hook member 18 provided in the second corner region 19 on an inner side of the outer shell 1. The first and the second hook members 16,18 are intended to be attached to a hook attachment zone 20 (shown in Fig. 1B) provided on the outer surface 4 of the outer shell 1 to form a waist opening.

Fig. 3 illustrates an outer shell 1 according to the present disclosure provided with an absorbent insert 2. The outer shell 1 comprises a front pocket 8 arranged in a front region 5 of the outer shell 1 on the inner surface 3 thereof and a rear pocket 9 arranged on the inner surface 3 of the outer shell in a rear region 7 thereof. The front pocket 8 receives a front section 2a of the absorbent insert 2 and the rear pocket 9 receives a rear section 2b of the absorbent insert 2.

The front pocket 8 comprises a front pocket elastic member 13 arranged in a front pocket transverse distal edge region 12 extending along the front pocket transverse distal edge 8a and the rear pocket 9 comprises a rear pocket elastic member 21 arranged in a rear pocket transverse distal edge region 15 extending along the rear pocket transverse distal edge 9a.

To releasably attach the absorbent insert 2 to the outer shell 1, the outer shell 1 comprises a front hook member 10 arranged in the front pocket 8 of the outer shell 1 and a rear hook member 11 arranged in the rear pocket 9 of the outer shell 1. The front and rear hook members 10,11 are releasably attached to a backsheet of the absorbent insert (not shown), such as for example a nonwoven/film layer backsheet. The front and rear hook members 10,11 may alternatively be attached to a loop material zone provided on the backsheet of the absorbent insert 2.

The front hook member 10 comprises a first front hook member part 10a and a second front hook member part 10b and the rear hook member 11 comprises a first rear hook member part 11a and a second rear hook member part 11b.

The outer shell 1 is of the open diaper type. The rear region 7 of the outer shell 1 furthermore comprises a first corner region 17 and a second corner region 19. The outer shell 1 comprises a first hook member 16 provided in the first corner region 17 and a second hook member 18 provided in the second corner region 19. The first and the second hook members 16,18 are intended to be arranged to face the outer surface 4 of the outer shell 1 during use and to be attached to a hook attachment zone 20 (shown in Fig. 1B) provided on the outer surface 4 of the outer shell 1 to form a waist opening.

The outer shell 1 comprises leg elastics 22,23 arranged along a respective longitudinal side edge of the outer shell 1 in the crotch region 6 and waist elastics 24 arranged along a rear transverse edge in the rear region 7 of the outer shell 1.

## Claims

1. A reusable outer shell (1) for a disposable absorbent insert (2) the outer shell (1) comprising an inner surface (3) intended to face the absorbent insert (2) and an outer surface (4) being opposite to the inner surface (3), the outer shell (1) having an extension in the longitudinal direction (L) and the transverse direction (T), the outer shell (1) comprising a front region (5), a crotch region (6) and a rear region (7), **characterized in that** the outer shell (1) comprises a front pocket (8) in the front region (5) and a rear pocket (9) in the rear region (7), the front pocket being adapted to receive a front portion (2a) of the absorbent insert (2) and the rear pocket being adapted to receive a rear portion (2b) of the absorbent insert (2), the front pocket (8) having a front pocket transverse distal edge (8a) adapted to extend over the front portion (2a) of the absorbent insert (2) and the rear pocket (9) having a rear pocket transverse distal edge (9a) adapted to extend over the rear portion (2b) of the absorbent insert (2), the front pocket has a front pocket length (l_{fp}), as measured along a longitudinal centerline (L_{c}) of the outer shell (1) from a front pocket transverse proximal edge (8b) to the front pocket transverse distal edge (8a) and the rear pocket has a rear pocket length (lᵣₚ), as measured along the longitudinal centerline (L_{c}) of the outer shell (1) and from a rear pocket transverse proximal edge (9b) to the rear pocket transverse distal edge (9a), and wherein the rear pocket length (lᵣₚ) is greater than the front pocket length (l_{fp}), **characterized in that** the reusable outer shell (1) comprises a front hook member (10) arranged on the inner surface (3) of the outer shell (1), inside the front pocket (8) and a rear hook member (11) arranged on the inner surface (3) of the outer shell (1) inside the rear pocket (9), wherein the front hook member (10) has a length (l_{f}) extending in the longitudinal direction (L) and a width (w_{f}) extending in the transverse direction (T) and the rear hook member (11) has a length (lᵣ) extending in the longitudinal direction (L) and a width (wᵣ) extending in the transverse direction (T).

2. The reusable outer shell (1) according to claim 1, wherein the rear pocket length (lᵣₚ) is at least 15% greater than the front pocket length (l_{fp}), optionally at least 25% greater or at least 40% greater.

3. The reusable outer shell (1) according to any one of the preceding claims, wherein the rear pocket (9) has a rear pocket transverse distal edge region (15) extending along the rear pocket transverse distal edge (9a) and wherein an elastic member (21) is arranged in the rear pocket transverse distal edge region (15) and extends in the transverse direction (T) and/or wherein the front pocket (8) has a front pocket transverse distal edge region (12) extending along the front pocket transverse distal edge (8a) and wherein an elastic member (13) is arranged in the front pocket transverse distal edge region (12) and extends in the transverse direction (T).

4. The reusable outer shell (1) according to one of the preceding claims, wherein the length (lᵣ) of the rear hook member (11) is greater than the length (l_{f}) of the front hook member (10).

5. The reusable outer shell (1) according to one of the preceding claims, wherein the length (lᵣ) of the rear hook member (11) is at least 15% greater than the length (l_{f}) of the front hook member (10), optionally at least 25% greater or at least 40% greater.

6. The reusable outer shell (1) according to one of the preceding claims, wherein the width (wᵣ) of the rear hook member (11) is within the range of from 50 mm to 200 mm, optionally within the range of from 75 mm to 150 mm.

7. The reusable outer shell (1) according to one of the preceding claims, wherein the width (w_{f}) of the front hook member (10) is within the range of from 50 mm to 200 mm, optionally within the range of from 75 mm to 150 mm.

8. The reusable outer shell (1) according to one of the preceding claims, wherein the rear hook member (11) comprises a first rear hook member part (11a) and a second rear hook member part (11b), the first rear hook member part (11a) and the second rear hook member part (11b) being spaced apart from each other with a distance (dᵣ) and arranged on a respective side of a longitudinal centerline (L_{c}) of the outer shell (1), the width (wᵣ) of the rear hook member (11) being measured at the greatest width between an outermost transverse edges (11a',11b') of the respective first rear hook member part (11a) and second rear hook member part (11b) and the length (lᵣ) of the rear hook member (11) being measured at the greatest length between a frontmost edge (11a",11b") of the first rear hook member part (11a) or the second rear hook member part (11b) and a rearmost edge (11a‴,11b‴) of the respective first rear hook member part (11a) or second rear hook member part (11b), optionally wherein the first rear hook member part (11a) has the same width and/or length as the second rear hook member part (11b), preferably wherein the first rear hook member part (11a) and the second rear hook member part (11b) cover from 25% to 100% of an area of the rear hook member (11), as measured by multiplying the length (lᵣ) of the rear hook member (11) with the width (wᵣ) of the rear hook member (11).

9. The reusable outer shell (1) according to claim 8, wherein the distance (dᵣ) between the first rear hook member part (11a) and the second rear hook member part (11b) corresponds to from 15% to 75% of the width (wᵣ) of the rear hook member (11).

10. The reusable outer shell (1) according to one of the preceding claims, wherein the front hook member (10) comprises a first front hook member part (10a) and a second front hook member part (10b), the first front hook member part (10a) and the second front hook member part (10b) being spaced apart from each other with a distance (d_{f}) and being arranged on a respective side of a longitudinal centerline (L_{c}) of the outer shell (1), the width (w_{f}) of the front hook member (10) being measured at the greatest width between the outermost transverse edges (10a',10b') of the respective first front hook member part (10a) and second front hook member part (10b) and the length (l_{f}) of the front hook member (10) being measured at the greatest length between a frontmost edge (10a",10b") of the first rear hook member part (11a) or the second rear hook member part (10b) and a rearmost edge (10a‴,10b‴) of the first front hook member part (10a) and second front hook member part (10b), optionally wherein the first front hook member part (10a) has the same width and/or length as the second front hook member part (10b).

11. The reusable outer shell (1) according to claim 10, wherein the distance (d_{f}) between the first front hook member part (10a) and the second front hook member part (10b) corresponds to from 15% to 75% of the width (w_{f}) of the front hook member (10).

12. The reusable outer shell (1) according to claim 10 or 11, wherein the first front hook member part (10a) and the second front hook member part (10b) cover from 25% to 100% of an area of the front hook member (10), as measured by multiplying the length (l_{f}) of the front hook member (10) with the width (w_{f}) of the front hook member (10).

13. The reusable outer shell (1) according to one of the preceding claims, wherein the rear hook member (11) has a width to length ratio (wᵣ/lᵣ) within the range of from 0.7:1 to 20:1.

14. The reusable outer shell (1) according to one of the preceding claims, wherein the front hook member (10) has a width to length ratio (w_{f}/l_{f}) of from 1:1 to 40:1.

15. The reusable outer shell (1) according to any one of the preceding claims, wherein the outer shell (1) in the rear region (7) comprises a first hook member (16) provided in a first rear transverse corner region (17) on an inner surface (3) of the outer shell (1) and a second hook member (18) is provided in a second rear transverse corner region (19) on the inner surface (3) of the outer shell (1), the outer shell (1) comprises a hook attachment zone (20) on the outer surface (4) in the front region (5) of the outer shell (1), the first and second hook members (16,18) being adapted to be attached to the hook attachment zone (20) to form a waist opening.

## Patentansprüche

1. Wiederverwendbare Außenhülle (1) für einen saugfähigen Einwegeinsatz (2), wobei die Außenhülle (1) eine Innenfläche (3), die dem saugfähigen Einsatz (2) zugewandt sein soll, und eine Außenfläche (4) umfasst, die der Innenfläche (3) gegenüberliegt, wobei die Außenhülle (1) eine Ausdehnung in der Längsrichtung (L) und der Querrichtung (T) aufweist, wobei die Außenhülle (1) einen vorderen Bereich (5), einen Schrittbereich (6) und einen hinteren Bereich (7) umfasst, **dadurch gekennzeichnet, dass** die Außenhülle (1) im vorderen Bereich (5) eine Vordertasche (8) und im hinteren Bereich (7) eine Hintertasche (9) umfasst, wobei die Vordertasche zur Aufnahme eines vorderen Abschnitts (2a) des saugfähigen Einsatzes (2) angepasst ist, und die Hintertasche zur Aufnahme eines hinteren Abschnitts (2b) des saugfähigen Einsatzes (2) angepasst ist, wobei die Vordertasche (8) eine distale Querkante der Vordertasche (8a) aufweist, die so angepasst ist, dass sie sich über den vorderen Abschnitt (2a) des saugfähigen Einsatzes (2) erstreckt, und die Hintertasche (9) eine distale Querkante der Hintertasche (9a) aufweist, die so angepasst ist, dass sie sich über den hinteren Abschnitt (2b) des saugfähigen Einsatzes (2) erstreckt, wobei die Vordertasche eine Vordertaschenlänge (lf_{P}) aufweist, gemessen entlang einer Längsmittellinie (L_{c}) der Außenhülle (1) von einer proximalen Querkante der Vordertasche (8b) bis zur distalen Querkante der Vordertasche (8a) und die Hintertasche eine Hintertaschenlänge (lᵣₚ) aufweist, gemessen entlang der Längsmittellinie (L_{c}) der Außenhülle (1) und von einer proximalen Querkante der Hintertasche (9b) bis zur distalen Querkante der Hintertasche (9a), und wobei die Hintertaschenlänge (lᵣₚ) größer ist als die Vordertaschenlänge (l_{fp}), **dadurch gekennzeichnet, dass** die wiederverwendbare Außenhülle (1) ein vorderes Hakenelement (10) umfasst, das an der Innenfläche (3) der Außenhülle (1) innerhalb der Vordertasche (8) angeordnet ist, und ein hinteres Hakenelement (11) umfasst, das an der Innenfläche (3) der Außenhülle (1) innerhalb der Hintertasche (9) angeordnet ist, wobei das vordere Hakenelement (10) eine Länge (l_{f}) aufweist, die sich in der Längsrichtung (L) erstreckt und eine Breite (w_{f}) aufweist, die sich in der Querrichtung (T) erstreckt, und das hintere Hakenelement (11) eine Länge (lᵣ) aufweist, die sich in der Längsrichtung (L) erstreckt und eine Breite (wᵣ) aufweist, die sich in der Querrichtung (T) erstreckt.

2. Wiederverwendbare Außenhülle (1) nach Anspruch 1, wobei die Hintertaschenlänge (lᵣₚ) mindestens 15 % größer ist als die Vordertaschenlänge (l_{fp}), optional mindestens 25 % größer oder mindestens 40 % größer.

3. Wiederverwendbare Außenhülle (1) nach einem der vorstehenden Ansprüche, wobei die Hintertasche (9) einen distalen Querkantenbereich der Hintertasche (15) aufweist, der sich entlang der distalen Querkante der Hintertasche (9a) erstreckt, und wobei ein elastisches Element (21) im distalen Querkantenbereich der Hintertasche (15) angeordnet ist und sich in der Querrichtung (T) erstreckt, und/oder wobei die Vordertasche (8) einen distalen Querkantenbereich der Vordertasche (12) aufweist, der sich entlang der distalen Querkante der Vordertasche (8a) erstreckt, und wobei ein elastisches Element (13) im distalen Querkantenbereich der Vordertasche (12) angeordnet ist und sich in der Querrichtung (T) erstreckt.

4. Wiederverwendbare Außenhülle (1) nach einem der vorstehenden Ansprüche, wobei die Länge (lᵣ) des hinteren Hakenelements (11) größer ist als die Länge (l_{f}) des vorderen Hakenelements (10).

5. Wiederverwendbare Außenhülle (1) nach einem der vorstehenden Ansprüche, wobei die Länge (lᵣ) des hinteren Hakenelements (11) mindestens 15 % größer ist als die Länge (l_{f}) des vorderen Hakenelements (10), optional mindestens 25 % größer oder mindestens 40 % größer.

6. Wiederverwendbare Außenhülle (1) nach einem der vorstehenden Ansprüche, wobei die Breite (wᵣ) des hinteren Hakenelements (11) im Bereich von 50 mm bis 200 mm, optional im Bereich von 75 mm bis 150 mm liegt.

7. Wiederverwendbare Außenhülle (1) nach einem der vorstehenden Ansprüche, wobei die Breite (w_{f}) des vorderen Hakenelements (10) im Bereich von 50 mm bis 200 mm, optional im Bereich von 75 mm bis 150 mm liegt.

8. Wiederverwendbare Außenhülle (1) nach einem der vorstehenden Ansprüche, wobei das hintere Hakenelement (11) einen ersten hinteren Hakenelementteil (11a) und einen zweiten hinteren Hakenelementteil (11b) umfasst, wobei das erste hintere Hakenelementteil (11a) und das zweite hintere Hakenelementteil (11b) mit einem Abstand (dᵣ) voneinander beabstandet sind und auf einer jeweiligen Seite einer Längsmittellinie (L_{c}) der Außenhülle (1) angeordnet sind, die Breite (wᵣ) des hinteren Hakenelements (11) an der größten Breite zwischen den äußersten Querkanten (11a', 11b') des jeweiligen ersten hinteren Hakenelementteils (11a) und zweiten hinteren Hakenelementteils (11b) gemessen wird, und die Länge (lᵣ) des hinteren Hakenelements (11) an der größten Länge zwischen einer vordersten Kante (11a", 11b") des ersten hinteren Hakenelementteils (11a) oder des zweiten hinteren Hakenelementteils (11b) und einer hintersten Kante (11a‴, 11b‴) des jeweiligen ersten hinteren Hakenelementteils (11a) oder zweiten hinteren Hakenelementteils (11b) gemessen wird, optional wobei das erste hintere Hakenelementteil (11a) die gleiche Breite und/oder Länge aufweist wie das zweite hintere Hakenelementteil (11b), vorzugsweise wobei der erste hintere Hakenelementteil (11a) und der zweite hintere Hakenelementteil (11b) eine Fläche von 25 % bis 100 % des hinteren Hakenelements (11) abdecken, gemessen durch Multiplikation der Länge (lᵣ) des hinteren Hakenelements (11) mit der Breite (wᵣ) des hinteren Hakenelements (11).

9. Wiederverwendbare Außenhülle (1) nach Anspruch 8, wobei der Abstand (dᵣ) zwischen dem ersten hinteren Hakenelementteil (11a) und dem zweiten hinteren Hakenelementteil (11b) 15 % bis 75 % der Breite (wᵣ) des hinteren Hakenelements (11) entspricht.

10. Wiederverwendbare Außenhülle (1) nach einem der vorstehenden Ansprüche, wobei das vordere Hakenelement (10) ein erstes vorderes Hakenelementteil (10a) und ein zweites vorderes Hakenelementteil (10b) umfasst, wobei das erste vordere Hakenelementteil (10a) und das zweite vordere Hakenelementteil (10b) mit einem Abstand (d_{f}) voneinander beabstandet sind und auf einer jeweiligen Seite einer Längsmittellinie (L_{c}) der Außenhülle (1) angeordnet sind, die Breite (w_{f}) des vorderen Hakenelements (10) an der größten Breite zwischen den äußersten Querkanten (10a', 10b') des jeweiligen ersten vorderen Hakenelementteils (10a) und zweiten vorderen Hakenelementteils (10b) gemessen wird, und die Länge (l_{f}) des vorderen Hakenelements (10) an der größten Länge zwischen einer vordersten Kante (10a", 10b") des ersten hinteren Hakenelementteils (11a) oder des zweiten hinteren Hakenelementteils (10b) und einer hintersten Kante (10a‴, 10b‴) des ersten vorderen Hakenelementteils (10a) und des zweiten vorderen Hakenelementteils (10b) gemessen wird, optional wobei das erste vordere Hakenelementteil (10a) die gleiche Breite und/oder Länge aufweist wie das zweite vordere Hakenelementteil (10b).

11. Wiederverwendbare Außenhülle (1) nach Anspruch 10, wobei der Abstand (d_{f}) zwischen dem ersten vorderen Hakenelementteil (10a) und dem zweiten vorderen Hakenelementteil (10b) 15 % bis 75 % der Breite (w_{f}) des vorderen Hakenelements (10) entspricht.

12. Wiederverwendbare Außenhülle (1) nach Anspruch 10 oder 11, wobei das erste vordere Hakenelementteil (10a) und das zweite vordere Hakenelementteil (10b) eine Fläche von 25 % bis 100 % des vorderen Hakenelements (10) abdecken, gemessen durch Multiplikation der Länge (lᵣ) des vorderen Hakenelements (10) mit der Breite (w_{f}) des vorderen Hakenelements (10).

13. Wiederverwendbare Außenhülle (1) nach einem der vorstehenden Ansprüche, wobei das hintere Hakenelement (11) ein Breiten-Längen-Verhältnis (wᵣ/lᵣ) im Bereich von 0,7:1 bis 20:1 aufweist.

14. Wiederverwendbare Außenhülle (1) nach einem der vorstehenden Ansprüche, wobei das vordere Hakenelement (10) ein Breiten-Längen-Verhältnis (w_{f}/l_{f}) von 1:1 bis 40:1 aufweist.

15. Wiederverwendbare Außenhülle (1) nach einem der vorstehenden Ansprüche, wobei die Außenhülle (1) im hinteren Bereich (7) ein erstes Hakenelement (16) umfasst, das in einem ersten hinteren querverlaufenden Eckbereich (17) auf einer Innenfläche (3) der Außenhülle (1) vorgesehen ist, und ein zweites Hakenelement (18) in einem zweiten hinteren querverlaufenden Eckbereich (19) auf der Innenfläche (3) der Außenhülle (1) vorgesehen ist, die Außenhülle (1) eine Hakenbefestigungszone (20) auf der Außenfläche (4) im vorderen Bereich (5) der Außenhülle (1) umfasst, wobei das erste und das zweite Hakenelement (16, 18) so angepasst sind, dass sie an der Hakenbefestigungszone (20) befestigt werden, um eine Taillenöffnung zu bilden.

## Revendications

1. Enveloppe extérieure réutilisable (1) pour un insert absorbant jetable (2), l'enveloppe extérieure (1) comprenant une surface intérieure (3) destinée à faire face à l'insert absorbant (2) et une surface extérieure (4) opposée à la surface intérieure (3), l'enveloppe extérieure (1) présentant une extension dans la direction longitudinale (L) et la direction transversale (T), l'enveloppe extérieure (1) comprenant une région avant (5), une région d'entrejambe (6) et une région arrière (7), **caractérisée en ce que** l'enveloppe extérieure (1) comprend une poche avant (8) dans la région avant (5) et une poche arrière (9) dans la région arrière (7), la poche avant étant adaptée pour recevoir une partie avant (2a) de l'insert absorbant (2) et la poche arrière étant adaptée pour recevoir une partie arrière (2b) de l'insert absorbant (2), la poche avant (8) présentant un bord distal transversal de poche avant (8a) adapté pour s'étendre sur la partie avant (2a) de l'insert absorbant (2) et la poche arrière (9) présentant un bord distal transversal de poche arrière (9a) adapté pour s'étendre sur la partie arrière (2b) de l'insert absorbant (2), la poche avant présente une longueur de poche avant (l_{fp}), telle que mesurée le long d'une ligne médiane longitudinale (L_{c}) de l'enveloppe extérieure (1) depuis un bord proximal transversal de poche avant (8b) jusqu'au bord distal transversal de poche avant (8a) et la poche arrière présente une longueur de poche arrière (lᵣₚ), telle que mesurée le long de la ligne médiane longitudinale (L_{c}) de l'enveloppe extérieure (1) et depuis un bord proximal transversal de poche arrière (9b) jusqu'au bord distal transversal de poche arrière (9a), et dans laquelle la longueur de poche arrière (lᵣₚ) est supérieure à la longueur de poche avant (l_{fp}), **caractérisée en ce que** l'enveloppe extérieure réutilisable (1) comprend un élément à crochets avant (10) agencé sur la surface intérieure (3) de l'enveloppe extérieure (1), à l'intérieur de la poche avant (8) et un élément à crochets arrière (11) agencé sur la surface intérieure (3) de l'enveloppe extérieure (1) à l'intérieur de la poche arrière (9), dans laquelle l'élément à crochets avant (10) présente une longueur (lᵣ) s'étendant dans la direction longitudinale (L) et une largeur (w_{f}) s'étendant dans la direction transversale (T) et l'élément à crochets arrière (11) présente une longueur (lᵣ) s'étendant dans la direction longitudinale (L) et une largeur (wᵣ) s'étendant dans la direction transversale (T).

2. Enveloppe extérieure réutilisable (1) selon la revendication 1, dans laquelle la longueur de poche arrière (lᵣₚ) est au moins 15 % supérieure à la longueur de poche avant (l_{fp}), facultativement au moins 25 % supérieure ou au moins 40 % supérieure.

3. Enveloppe extérieure réutilisable (1) selon l'une quelconque des revendications précédentes, dans laquelle la poche arrière (9) présente une région de bord distal transversal de poche arrière (15) s'étendant le long du bord distal transversal de poche arrière (9a) et dans laquelle un élément élastique (21) est agencé dans la région de bord distal transversal de poche arrière (15) et s'étend dans la direction transversale (T) et/ou dans laquelle la poche avant (8) présente une région de bord distal transversal de poche avant (12) s'étendant le long du bord distal transversal de poche avant (8a) et dans laquelle un élément élastique (13) est agencé dans la région de bord distal transversal de poche avant (12) et s'étend dans la direction transversale (T).

4. Enveloppe extérieure réutilisable (1) selon l'une des revendications précédentes, dans laquelle la longueur (lᵣ) de l'élément à crochets arrière (11) est supérieure à la longueur (l_{f}) de l'élément à crochets avant (10).

5. Enveloppe extérieure réutilisable (1) selon l'une des revendications précédentes, dans laquelle la longueur (lᵣ) de l'élément à crochets arrière (11) est au moins 15 % supérieure à la longueur (lᵣ) de l'élément à crochets avant (10), facultativement au moins 25 % supérieure ou au moins 40 % supérieure.

6. Enveloppe extérieure réutilisable (1) selon l'une des revendications précédentes, dans laquelle la largeur (wᵣ) de l'élément à crochets arrière (11) est comprise dans une plage allant de 50 mm à 200 mm, facultativement dans une plage allant de 75 mm à 150 mm.

7. Enveloppe extérieure réutilisable (1) selon l'une des revendications précédentes, dans laquelle la largeur (w_{f}) de l'élément à crochet avant (10) est comprise dans une plage allant de 50 mm à 200 mm, facultativement dans une plage allant de 75 mm à 150 mm.

8. Enveloppe extérieure réutilisable (1) selon l'une des revendications précédentes, dans laquelle l'élément à crochets arrière (11) comprend une première partie d'élément à crochets arrière (11a) et une deuxième partie d'élément à crochets arrière (11b), la première partie d'élément à crochets arrière (11a) et la deuxième partie d'élément à crochets arrière (11b) étant espacées l'une de l'autre d'une distance (dᵣ) et agencées sur un côté respectif d'une ligne médiane longitudinale (L_{c}) de l'enveloppe extérieure (1), la largeur (wᵣ) de l'élément à crochets arrière (11) étant mesurée à la plus grande largeur entre des bords transversaux les plus extérieurs (11a', 11b') de la première partie d'élément à crochets arrière (11a) et de la deuxième partie d'élément à crochets arrière (11b) respectives et la longueur (lᵣ) de l'élément à crochets arrière (11) étant mesurée à la plus grande longueur entre un bord le plus avant (11a", 11b") de la première partie d'élément à crochets arrière (11a) ou de la deuxième partie d'élément à crochets arrière (11b) et un bord le plus arrière (11a‴, 11b‴) de la première partie d'élément à crochets arrière (11a) ou de la deuxième partie d'élément à crochets arrière (11b) respective, facultativement dans laquelle la première partie d'élément à crochets arrière (11a) présente la même largeur et/ou longueur que la deuxième partie d'élément à crochets arrière (11b), de préférence dans laquelle la première partie d'élément à crochets arrière (11a) et la deuxième partie d'élément à crochets arrière (11b) couvrent de 25 % à 100 % d'une surface de l'élément à crochets arrière (11), telle que mesurée en multipliant la longueur (lᵣ) de l'élément à crochets arrière (11) par la largeur (wᵣ) de l'élément à crochets arrière (11).

9. Enveloppe extérieure réutilisable (1) selon la revendication 8, dans laquelle la distance (dᵣ) entre la première partie d'élément à crochets arrière (11a) et la deuxième partie d'élément à crochets arrière (11b) correspond à de 15 % à 75 % de la largeur (wᵣ) de l'élément à crochets arrière (11).

10. Enveloppe extérieure réutilisable (1) selon l'une des revendications précédentes, dans laquelle l'élément à crochets avant (10) comprend une première partie d'élément à crochets avant (10a) et une deuxième partie d'élément à crochets avant (10b), la première partie d'élément à crochets avant (10a) et la deuxième partie d'élément à crochets avant (10b) étant espacées l'une de l'autre d'une distance (d_{f}) et étant agencées sur un côté respectif d'une ligne médiane longitudinale (L_{c}) de l'enveloppe extérieure (1), la largeur (w_{f}) de l'élément à crochets avant (10) étant mesurée à la plus grande largeur entre les bords transversaux les plus extérieurs (10a', 10b') de la première partie d'élément à crochets avant (10a) et de la deuxième partie d'élément à crochets avant (10b) respectives et la longueur (l_{f}) de l'élément à crochets avant (10) étant mesurée à la plus grande longueur entre un bord le plus avant (10a", 10b") de la première partie d'élément à crochets arrière (11a) ou de la deuxième partie d'élément à crochets arrière (10b) et un bord le plus arrière (10a'", 10b"') de la première partie d'élément à crochets avant (10a) et de la deuxième partie d'élément à crochets avant (10b), facultativement dans laquelle la première partie d'élément à crochets avant (10a) présente la même largeur et/ou longueur que la deuxième partie d'élément à crochets avant (10b).

11. Enveloppe extérieure réutilisable (1) selon la revendication 10, dans laquelle la distance (dᵣ) entre la première partie d'élément à crochets avant (10a) et la deuxième partie d'élément à crochets avant (10b) correspond à de 15 % à 75 % de la largeur (w_{f}) de l'élément à crochets avant (10).

12. Enveloppe extérieure réutilisable (1) selon la revendication 10 ou 11, dans laquelle la première partie d'élément à crochets avant (10a) et la deuxième partie d'élément à crochets avant (10b) couvrent de 25 % à 100 % d'une surface de l'élément à crochets avant (10), telle que mesurée en multipliant la longueur (l_{f}) de l'élément à crochets avant (10) par la largeur (w_{f}) de l'élément à crochets avant (10).

13. Enveloppe extérieure réutilisable (1) selon l'une des revendications précédentes, dans laquelle l'élément à crochets arrière (11) présente un rapport largeur sur longueur (wᵣ/lᵣ) compris dans une plage allant de 0,7:1 à 20:1.

14. Enveloppe extérieure réutilisable (1) selon l'une des revendications précédentes, dans laquelle l'élément à crochets avant (10) présente un rapport largeur sur longueur (wᵣ/lᵣ) compris dans une plage allant de 1:1 à 40:1.

15. Enveloppe extérieure réutilisable (1) selon l'une quelconque des revendications précédentes, dans laquelle l'enveloppe extérieure (1) dans la région arrière (7) comprend un premier élément à crochets (16) prévu dans une première région de coin transversal arrière (17) sur une surface intérieure (3) de l'enveloppe extérieure (1) et un deuxième élément à crochets (18) est prévu dans une deuxième région de coin transversal arrière (19) sur la surface intérieure (3) de l'enveloppe extérieure (1), l'enveloppe extérieure (1) comprend une zone de fixation à crochets (20) sur la surface extérieure (4) dans la région avant (5) de l'enveloppe extérieure (1), les premier et deuxième éléments à crochets (16, 18) étant adaptés pour être fixés à la zone de fixation à crochets (20) pour former une ouverture de taille.
